(19)

**Europäisches Patentamt**
**European Patent Office**
**Office européen des brevets**

(11)  **EP 4 137 585 A1**

(12)  **EUROPEAN PATENT APPLICATION**

(43) Date of publication:
**22.02.2023  Bulletin 2023/08**

(21) Application number: **21382772.8**

(22) Date of filing: **20.08.2021**

(51) International Patent Classification (IPC):
***C12Q 1/6886*** (2018.01)

(52) Cooperative Patent Classification (CPC):
**C12Q 1/6886;** C12Q 2600/106; C12Q 2600/158

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
- **Institut D'Investigacions Biomèdiques August
  Pi I Sunyer - IDIBAPS
  08036 Barcelona (ES)**
- **Universitat de Barcelona
  08028 Barcelona (ES)**
- **Hospital Clínic de Barcelona
  08036 Barcelona (ES)**
- **Consejo Superior de Investigaciones Científicas
  (CSIC)
  28006 Madrid (ES)**
- **Institució Catalana De Recerca I
  Estudis Avançats (ICREA)
  08010 Barcelona (ES)**

(72) Inventors:
- **MAUREL SANTASUSANA, Joan
  BARCELONA (ES)**
- **CASCANTE SERRATOSA, Marta
  BARCELONA (ES)**
- **PEDROSA EGUILAZ, Leire
  BARCELONA (ES)**
- **FOGUET COLL, Carles
  BARCELONA (ES)**
- **THOMSON OKATSU, Timothy
  BARCELONA (ES)**
- **POSTIGO, Antonio
  BARCELONA (ES)**
- **BENITEZ, Daniel
  BARCELONA (ES)**
- **CAMPS POLO, Jordi
  BARCELONA (ES)**

(74) Representative: **ZBM Patents - Zea, Barlocci &
Markvardsen
Rambla Catalunya, 123
08008 Barcelona (ES)**

(54)  **CANCER INFORMATIVE BIOMARKER SIGNATURE**

(57)  The present invention provides a method for deciding a check-point inhibitor (ICI)-based therapy, for a patient suffering from cancer, the method comprising the steps: a) measuring the expression level of the following markers: TGFB1, ZEB1, FAP, ZEB2, GLUL, ENTPD1, GOT1, LDHA, TWIST1, and GLS, in an isolated cancerous biological sample from the patient; b) determining which markers are differentially expressed; and c) classifying the sample, depending on the markers differentially expressed, in one of the following clusters: cluster 1: when ZEB1, ZEB2, ENTPD1, FAP and GLUL are upregulated; cluster 2: when GLS is upregulated; and LDHA, GOT1, ZEB1, ZEB2 and ENTPD1 are downregulated; and cluster 3: when GOT1 is upregulated and TGFB1 and TWIST1 are downregulated.

**EP 4 137 585 A1**

**Description**

**Technical Field**

The present invention relates to the field of cancer, particularly of the immunotherapy and prognosis. In particular, the present invention provides methods and kits for personalizing combined therapeutic strategies with check-point inhibitors (ICI) in patients suffering from cancer, as well as prognostic methods, all of them based on an immune-metabolic marker signature.

**Background Art**

The use of programmed cell death ligand (PD-L1) immunohistochemistry (IHC) as a predictive biomarker for immune check-point inhibitors (ICI) has been confounded by multiple unresolved issues (e.g. variable detection antibodies, differing IHC cutoffs, tissue preparation and processing variability).

PD-L1 IHC expression in tumoral cells or combined positive score (CPS) that evaluate PD-L1 expression both in epithelial tumoral and immune cells, has been assessed prospectively in randomized clinical trials across tumor types to improve ICI accuracy, with controversial results (Shitara K *et al.,* 2020).

Up to now, immune markers and tumor mutational burden provide show modest predictive accuracies of immune -check-point inhibitors (ICI) efficacy.

Therefore, there is still the need of markers providing predictive information to appropriately manage anticancer therapy.

**Summary of Invention**

The present inventors have found a panel based on 10 biomarkers, particularly on GLS, TGFB1, TWIST1, GOT1, LDHA, GLUL, FAP, ZEB1, ZEB2, and ENTPD1, which can provide accurate information of the cancer and provide predictive information about therapeutic approaches.

As it is shown below, the inventors found, using a first set of 75 CRC samples, that tumor samples could be classified in three well-differentiated clusters, depending on the particular expression profile determined for these 10 markers: Cluster 1, wherein ZEB1, ZEB2, ENTPD1, FAP and GLUL are up-regulated; Cluster 2, wherein GLS is upregulated and LDHA, GOT1, ZEB1, ZEB2 and ENTPD1 are downregulated; and Cluster 3, wherein GOT1 is upregulated and TGFB1 and TWIST1 are downregulated.

Advantageously, the above identified Clusters 1 to 3 exhibited well-differentiated metabolic/immunologic profiles.

This allows to classify cancer samples in three main groups on the basis of the main metabolic genes that are affected in the reprogramming of cancer cells. Mainly, the cancer samples falling in Cluster 1 were consistent with a cancer cell mesenchymal immunosuppressive subtype; the cancer samples falling in Cluster 2 were consistent with an epithelial phenotype without glycolysis activation; and the cancer samples falling in Cluster 3 were consistent with an epithelial phenotype with glycolysis activation:

The above means that the tumor biopsies could exhibit three main different metabolic phenotypes based on the differential expression of the above metabolic markers. This is indicative that the cancer cells would metabolically adapt in three main ways, based on different factors such as genetic drivers, nutritional status and metabolic crosstalk with tumor-infiltrating immune cells, and other stromal components, in the tumor microenvironment.

This metabolic and immunologic differences between the three clusters not only allow to precisely classify the patient suffering from cancer in one of the above clusters, but also, depending on the cluster wherein they are classified, a well-defined candidate therapeutic approach can be precisely defined by the physician, based on the use of a check-point inhibitor therapy.

The above information is of great importance at the moment of designing the best candidate protocol to efficiently treat the patient suffering from cancer, improving the management of the therapies but, specially, gaining time, something critical in the outcome of this disease and variants thereof.

Thus, if the patient is classified as falling in Cluster 1, this will be indicative that they are candidate to be sensitive to a combined therapy based on ICI and one or more of: GFPT2 inhibitors, through depleting glycan enriched tumor microenvironment. Additional putative combined therapies could be immune-checkpoint blockade combined with either, TGF-b inhibitors, CD39 inhibitors, IDO1-AHR inhibitors or MCTs (lactate transporters) inhibitors.

If the patient is classified as falling in Cluster 2, this will be indicative that they are candidate to be sensitive to a combined therapy based on ICI and one or more of: drugs targeting citrate transporters, to interfere with cancer cell uptake of lipogenesis precursors and lipid synthesis, to interfere with macrophage-cancer cells metabolic interdependence. Inhibitors of gluconeogenesis can enhance ICI activity. In addition, inhibitor of autophagy could also restore MCH levels and immune sensitivity in combination with ICI.

If the patient is classified as falling in Cluster 3, this will be indicative that they are candidate to be sensitive to a combined therapy based on ICI and one or more of: glucose transporter inhibitors, amino-acid transporter inhibitors, CDK4 inhibitors, 6GPD inhibitors, and doble blocking strategies (to minimize ROS production and anti-oxidant mechanisms).

The inventors checked the versatility of the 10-marker panel of the invention in an extensive cohort of 4200 samples of 11 different tumors: again, all of them could be appropriately classified in one of the above-identified three clusters. Not only that, but also, the inventors performed an extensive expression profile analysis of the 4200 cancer samples classified in each one of the clusters. They found that further markers were dysregulated but all of these additional markers complement the metabolic/immunological interactions described in the invention.

Thus, the cancer samples falling in Cluster 1, consistent with a cancer cell mesenchymal immunosuppressive subtype use pyruvate to produce lactate instead of being oxidized in the tricarboxylic acid (TCA) cycle and these CAFs through macropinocytosis and autophagy obtain amino acids and glutamine to sustain their needs and also to release to the microenvironment supplying with alternative carbon and nitrogen sources the TCA cycle in cancer cells. This complex and wasting-energy metabolic rewiring, facilitate extracellular acidification (high lactate). In addition, this cluster show enhanced metabolic up-regulation of key enzymes in Kynurenine biosynthetic pathway, PD-1 and PD-L1 glycosylation due to increased HBP and CD39 expression contributing all to high immunosuppression.

Cancer samples falling in Cluster 2 are consistent with an epithelial phenotype without glycolysis activation: an upregulation of lipid transporters, gluconeogenesis, and glutamine and fatty acid synthesis, a metabolic reprogramming previously described in glucose and glutamine depleted microenvironments, which is indicative of a metabolic interdependence between TAM and cancer cells. Interestingly, our results unveil a cluster 2-specific upregulation of V-ATPase a key player in MHC lysosomal degradation and genes implicated in canonical autophagy pathway, that contributes to loss of sensitivity to TNF$\alpha$ and INF$\gamma$ and it's characterized by the presence of bystander CD8+, CD39-.

Cancer samples falling in Cluster 3 are consistent with an epithelial phenotype with glycolysis activation: upregulation of several proteins involved in cell cycle as well as upregulation of key metabolic players that provide metabolic advantage to these tumor cells for anchorage-independent growth and metabolic flexibility to adapt to redox stress, oxygen, or nutrient-altered environments by means of overexpression of key genes on folate, one-carbon, pentose phosphate, OXPHOS and glycolysis genes that provide ability for ROS neutralization and reductive carboxylation from glutamine.

Therefore, the inventors have found that with a panel of just 10 markers the physician can get a very precise picture of the nature/phenotype of the cancer in terms of metabolism and immunologic status. And this represents a great advance in the management of therapeutic approaches for the treatment of cancer.

Until the present invention the markers of the invention had not been provided as useful in providing such powerful predictive therapeutic information.

Thus, in a first aspect the present invention provides a method for deciding a check-point inhibitor (ICI)-based therapy, the method comprising the steps:

> a) measuring the expression level of the following markers: TGFB1, ZEB1, FAP, ZEB2, GLUL, ENTPD1, GOT1, LDHA, TWIST1, and GLS, in an isolated cancerous biological sample from the patient;

> b) determining which markers are differentially expressed; and

> c) classifying the sample, depending on the markers differentially expressed, in one of the following clusters:

>> cluster 1: when ZEB1, ZEB2, ENTPD1, FAP and GLUL are up-regulated;

>> cluster 2: when GLS is upregulated; and LDHA, GOT1, ZEB1, ZEB2 and ENTPD1 are downregulated; and

>> cluster 3: when GOT1 is upregulated and TGFB1 and TWIST1 are downregulated; wherein:

>>> when the sample is classified in "cluster 1" then it is indicative that the patient is candidate to benefit from a combined therapy comprising a check-point inhibitor (ICI) therapy and one or more of: GFPT2 inhibitors, through depleting glycan enriched tumor microenvironment; TGF-b inhibitors, CD39 inhibitors, IDO1-AHR inhibitors or MCTs (lactate transporters) inhibitors;

>>> when the sample is classified in "Cluster 2", then it is indicative that the patient is candidate to benefit from a combined therapy comprising ICI therapy and one or more of: drugs targeting citrate transporters, to interfere with cancer cell uptake of lipogenesis precursors and lipid synthesis, to interfere with macrophage-cancer cells metabolic interdependence, inhibitors of gluconeogenesis or inhibitors of autophagy;

when the sample is classified in "Cluster 3", then it is indicative that the patient is candidate to benefit from a combined therapy comprising ICI therapy with one or more of: glucose transporter inhibitors, amino-acid transporter inhibitors, CDK4 inhibitors, 6GPD inhibitors, and doble blocking strategies (to minimize ROS production and intrinsic antioxidant mechanisms).

The set of 10 genes in the form of the three clusters defined above also provides valuable information about the patient suffering from cancer. As it is shown below, there was a poor prognosis when the patient sample was classified in Cluster 1, the overall survival being remarkably reduced. On the contrary, when the patient sample was classified in clusters 2 or 3, the prognosis was better.

Therefore, in a second aspect the present invention provides a method of determining the prognosis of a patient suffering from cancer:

a) measuring the expression level of the markers TGFB1, ZEB1, FAP, ZEB2, GLUL, ENTPD1, GOT1, LDHA, TWIST1, and GLS in a cancerous biological sample obtained from the patient; and

b) determining which markers are differentially expressed; and

c) classifying the sample, depending on the markers differentially expressed, in one of the following clusters:

cluster 1: when ZEB1, ZEB2, ENTPD1, FAP and GLUL are up-regulated;

cluster 2: when GLS is upregulated; and LDHA, GOT1, ZEB1, ZEB2 and ENTPD1 are downregulated; and

cluster 3: when GOT1 is upregulated and TGFB1 and TWIST1 are downregulated; wherein:
when the sample is classified in "cluster 1" then it is indicative that the patient has a bad prognosis (low OS);
whereas when the sample is classified in any of the clusters 2 or 3, the patient has a good prognosis (high OS).

In a final aspect the present invention provides a kit comprising means for determining the expression of the markers TGFB1, ZEB1, FAP, ZEB2, GLUL, ENTPD1, GOT1, LDHA, TWIST1, and GLS.

**Detailed description of the invention**

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The present invention provides methods for recommending therapeutic protocols based on check-point inhibitors (ICI)-based therapy in a subject suffering from cancer. Not only that, but also, the invention allows appropriate and concisely manage ICI therapy to effectively treat the disease.

In the present invention, the term "checkpoint inhibitor" (ICI) is understood as a form of cancer immunotherapy. The therapy targets immune checkpoints, key regulators of the immune system that when stimulated can dampen the immune response to an immunologic stimulus. In one embodiment of the methods of the invention, optionally in combination with any of the embodiments provided above or below, the checkpoint inhibitor inhibits a checkpoint protein selected from the group consisting of: cytotoxic T-lymphocyte antigen-4 (CTLA4), programmed cell death protein 1 (PD-1), PD-L1, PD-L2, B7-H3, B7-H4, herpesvirus entry mediator (HVEM), T cell membrane protein 3 (TIM3), galectin 9 (GAL9), lymphocyte activation gene 3 (LAG3), V-domain immunoglobulin (Ig)-containing suppressor of T-cell activation (VISTA), Killer-Cell Immunoglobulin-Like Receptor (KIR), B and T lymphocyte attenuator (BTLA), T cell immunoreceptor with Ig and ITIM domains (TIGIT), and combinations thereof. In one embodiment, optionally in combination with any of the embodiments provided above or below, the checkpoint inhibitor inhibits CTLA-4, PD-1 or PD-L1. In one embodiment, optionally in combination with any of the embodiments provided above or below, the checkpoint inhibitor inhibits CTLA-4 and is selected from Ipilimumab and Tremelimumab. In one embodiment, optionally in combination with any of the embodiments provided above or below, the checkpoint inhibitor inhibits PD-1 and is selected from Nivolumab, Pembrolizumab, Pidilizumab, lambrolizumab, and AMP-224. In one embodiment, optionally in combination with any of the embodiments provided above or below, the checkpoint inhibitor inhibits PD-L1 and is selected from BMS-936559, MEDI-4736, MPDL33280A, M1H1, Atezolizumab, Durvalumab and Avelumab.

In the present invention the terms "marker" and "biomarker" have the same meaning and are used interchangeably.

In the present invention, the term "sensitive to treatment with" means a condition (e.g., a neoplasm) that is responsive to an initial (and in some examples subsequent) therapy or treatment. For example, a condition (e.g., a neoplasm) that is statistically significantly responsive to an initial (and in some examples, subsequent) therapy or treatment. In an example, sensitivity refers to the responsiveness of a disease or symptom or progression thereof, such as the growth of a cancer, to an agent (such as a therapeutic agent) or combination of agents. For example, an increased (relative) sensitivity refers to a state in which a neoplasm is more responsive to a given therapy or therapeutic agent or treatment, as compared to a neoplasm that is not sensitive to the treatment.

In certain examples, sensitivity or responsiveness of a cancer/neoplasm can be assessed using any endpoint indicating a benefit to the subject, including, without limitation: (1) inhibition, to some extent, of neoplasm growth, including slowing down and complete growth arrest; (2) reduction in the number of neoplasm cells; (3) reduction in neoplasm size or volume; (4) inhibition (such as reduction, slowing down or complete stopping) of neoplasm cell infiltration into adjacent peripheral organs and/or tissues; (5) inhibition (such as reduction, slowing down or complete stopping) of metastasis; (6) enhancement of antineoplasm immune response, which may, but does not have to, result in the regression or rejection of the neoplasm; (7) relief, to some extent, of one or more symptoms associated with the neoplasm; (8) increase in the length of survival following treatment; and/or (9) decreased mortality at a given point of time following treatment.

In some examples, sensitivity of a cancer/neoplasm to treatment can be assessed before treatment to determine if the cancer/neoplasm will respond to the treatment. In further examples, sensitivity of a cancer/neoplasm to treatment can be assessed after treatment of the cancer/neoplasm to determine if the cancer/neoplasm is responding to the treatment. In some embodiments, sensitivity of a cancer/neoplasm to treatment can be assessed after initiation of treatment (for example, no more than 8 hours, no more than 12 hours, no more than 1 day, no more than 2 days, no more than 3 days, no more than 4 days, no more than 5 days, no more than 6 days, no more than 1 week, no more than 2 weeks, no more than 3 weeks or no more than 1 month, such as 8 hours, 12 hours, 1 day, 2 days, 3 days, 4 days, 5 days, six days, 1 week, 2 weeks, 3 weeks or 1 month, following initiation of treatment), to determine if the neoplasm is responding to the treatment. In some such embodiments, the neoplasm has a response that includes changes in gene expression that can be detected before a physical response (such as reduction of tumor burden) is detectable.

As used herein, "benefit" refers to drug effect and/or efficacy attributable to a drug treatment. For example, a drug benefit includes the impact of the drug on the likelihood of survival of a subject or patient, which can be expressed using overall survival and/or distant relapse-free survival. A "greater benefit" or "lesser benefit" may refer to the benefit of the therapy on the survival of patient compared to a lack of anthracycline therapy.

In one embodiment of the methods of the present invention, optionally in combination with any of the embodiments provided above or below, the test sample is a needle biopsy core, a surgical resection sample, or a body fluid sample (such as blood, serum, plasma, aspirates and urine). For example, these methods include obtaining a biopsy, which is optionally fractionated by cryostat sectioning to enrich diseases cells to about 80% of the total cell population. In certain embodiments, polynucleotides extracted from these samples may be amplified using techniques well known in the art. In one embodiment of the methods of the invention, optionally in combination with any of the embodiments provided above or below, the test sample is cancer tissue sample, particularly a paraffin-embedded tissue sample.

In the present invention the term "level of expression" means the amount of the marker either in the form of cDNA, RNA, protein or a fragment thereof. In one embodiment of the methods of the invention, optionally in combination with any of the embodiments provided above or below, it is measured the amount of RNA or cDNA. In one embodiment of the methods of the invention, optionally in combination with any of the embodiments provided above or below, the step of measuring the expression level comprises determining the amount of mRNA or long non-coding RNA.

Methods for quantitating mRNA are well known in the art. In some examples, the method utilizes RT-PCR. For example, extracted RNA can be reverse-transcribed using a GeneAmp® RNA PCR kit (Perkin Elmer, Calif., USA), following the manufacturer's instructions. In some embodiments, gene expression levels can be determined using a gene expression analysis technology that measure mRNA in solution. Examples of such gene expression analysis technologies include, but not limited to, RNAscope™, RT-PCR, Nanostring®, QuantiGene®, gNPA®., microarray, and sequencing. For example, methods of Nanostring use labeled reporter molecules, referred to as labeled "nanoreporters," that are capable of binding individual target molecules. Through the nanoreporters' label codes, the binding of the nanoreporters to target molecules results in the identification of the target molecules. Methods of Nanostring are described in U.S. Pat. No. 7,473,767.

PCR technology allows for the rapid generation of multiple copies of DNA sequences by providing 5' and 3' primers that hybridize to sequences present in an RNA or DNA molecule, and further providing free nucleotides and an enzyme (Taq polymerase) which fills in the complementary bases to the nucleotide sequence between the primers with the free nucleotides to produce a complementary strand of DNA. The enzyme will fill in the complementary sequences adjacent to the primers. If both the 5' primer and 3' primer hybridize to nucleotide sequences on the same small fragment of nucleic acid, exponential amplification of a specific double-stranded size product results. If only a single primer hybridizes to the nucleic acid fragment, linear amplification produces singlestranded products of variable length.

For example, TaqMan® RT-PCR can be performed using commercially available equipment. The system can include a thermocycler, laser, charge-coupled device (CCD) camera, and computer. The system amplifies samples in a 96-well format on a thermocycler. During amplification, laser-induced fluorescent signal is collected in realtime through fiber optics cables for all 96 wells, and detected at the CCD. The system includes software for running the instrument and for analyzing the data.

To minimize errors and the effect of sample-to-sample variation, RT-PCR can be performed using an internal standard. The ideal internal standard is expressed at a constant level among different tissues, and is unaffected by an experimental treatment. RNAs commonly used to normalize patterns of gene expression are mRNAs for the housekeeping genes GAPDH, β-actin, and 18S ribosomal RNA.

A variation of RT-PCR is real time quantitative RT-PCR, which measures PCR product accumulation through a dual-labeled fluorogenic probe (e.g., TaqMan® probe). Real time PCR is compatible both with quantitative competitive PCR, where internal competitor for each target sequence is used for normalization, and with quantitative comparative PCR using a normalization gene contained within the sample, or a housekeeping gene for RT-PCR. Instruments for carrying out quantitative PCR in microtiter plates are available from PE Applied Biosystems (Foster City, Calif.).

The steps of a representative protocol for quantitating gene expression level using fixed, paraffin-embedded tissues as the RNA source, including mRNA isolation, purification, primer extension and amplification are given in various published journal articles. Briefly, a representative process starts with cutting about 10 μm thick sections of paraffin-embedded neoplasm tissue samples or adjacent non-cancerous tissue. The RNA is then extracted, and protein and DNA are removed. Alternatively, RNA is isolated directly from a neoplasm sample or other tissue sample. After analysis of the RNA concentration, RNA repair and/or amplification steps can be included, if necessary, and RNA is reverse transcribed using gene specific promoters followed by RT-PCR.

PCR product may be detected by several well-known means. One way for detecting the presence of amplified DNA is to separate the PCR reaction material by gel electrophoresis and stain the gel with ethidium bromide in order to visual the amplified DNA if present. A size standard of the expected size of the amplified DNA is preferably run on the gel as a control.

In some instances, such as when unusually small amounts of RNA are recovered and only small amounts of cDNA are generated therefrom, it is desirable or necessary to perform a PCR reaction on the first PCR reaction product. That is, if difficult to detect quantities of amplified DNA are produced by the first reaction, a second PCR can be performed to make multiple copies of DNA sequences of the first amplified DNA. A nested set of primers are used in the second PCR reaction. The nested set of primers hybridize to sequences downstream of the 5' primer and upstream of the 3' primer used in the first reaction.

Alternatively, in another embodiment of the methods of the present invention, optionally in combination with any of the embodiments provided above or below, the determination of the expression level of the marker is performed by oligonucleotide hybridization technology. Oligonucleotide hybridization technology is well-known to those having ordinary skill in the art. Briefly, RNA or cDNA made from RNA from a sample is fixed, usually to filter paper or the like. The probes are added and maintained under conditions that permit hybridization only if the probes fully complement the fixed genetic material. The conditions are sufficiently stringent to wash off probes in which only a portion of the probe hybridizes to the fixed material. Detection of the probe on the washed filter indicates complementary sequences.

Probes useful in oligonucleotide assays are at least 18 nucleotides of complementary DNA and may be as large as a complete complementary sequence to cDNA marker. In some embodiments the probes are 30-200 nucleotides, preferably 40-100 nucleotides.

One having ordinary skill in the art, can design probes which are fully complementary to mRNA sequences but not genomic DNA sequences. Hybridization conditions can be routinely optimized to minimize background signal by non-fully complementary hybridization.

The probes are conveniently labelled either by radioactive (such as radiolabelled nucleotides) or non-radioactive detection systems.

Alternatively, in another embodiment of the methods of the present invention, optionally in combination with any of the embodiments provided above or below, the determination of the expression level of the marker comprises determining the amount of polypeptide marker. In another embodiment of the methods of the present invention, the determination of the polypeptide marker is performed by an immunoassay.

Immunoassays are commonly used to quantify the levels of proteins in cell samples, and many other immunoassay techniques are known in the art. The invention is not limited to a particular assay procedure, and therefore is intended to include both homogeneous and heterogeneous procedures. Exemplary immunoassays which can be conducted according to the invention include fluorescence polarisation immunoassay (FPIA), fluorescence immunoassay (FLA), enzyme immunoassay (EIA), nephelometric inhibition immunoassay (NIA), enzyme linked immunosorbent assay (ELISA), and radioimmunoassay (RIA). An indicator moiety, or label group, can be attached to the subject antibodies and is selected so as to meet the needs of various uses of the method which are often dictated by the availability of assay equipment and compatible immunoassay procedures. General techniques to be used in performing the various immunoassays noted above are known to those of ordinary skill in the art.

Other methods to quantify the level of a particular protein, or a protein fragment, or modified protein in a particular sample are based on flow-cytometric methods. Flow cytometry allows the identification of proteins on the cell surface as well as of intracellular proteins using fluorochrome labelled, protein specific antibodies or non-labelled antibodies in combination with fluorochrome labelled secondary antibodies. General techniques to be used in performing flow cytometric assays noted above are known to those of ordinary skill in the art.

The determination of the amount of polypeptide marker is performed by using an antibody or fragment thereof, such as Fab, F(ab)2, and Fv, which are capable of binding an epitope of the antigen.

Monoclonal antibodies which specifically bind to the polypeptide marker can be prepared using any technique which provides for the production of antibody molecules by continuous cell lines in culture. These techniques include, but are not limited to, the hybridoma technique, the human B cell hybridoma technique, and the EBV hybridoma technique.

In addition, techniques developed for the production of chimeric antibodies, the splicing of mouse antibody genes to human antibody genes to obtain a molecule with appropriate antigen specificity and biological activity can be used. Monoclonal and other antibodies can also be humanized to prevent a patient from mourning an immune response against the antibody when it is used therapeutically. Such antibodies may be sufficiently similar in sequence to human antibodies to be used directly in therapy or may require alteration of a few key residues. Sequence differences between rodent antibodies and human sequences can be minimized by replacing residues which differ from those in the human sequences by site directed mutagenesis of individual residues or by grating of entire complementarity determining regions.

Alternatively, techniques described for the production of single chain antibodies can be adapted using methods known in the art to produce single chain antibodies which specifically bind to polypeptide markers by using a DNA amplification method, such as PCR, using hybridoma cDNA as a template.

Single-chain antibodies can be mono- or bispecific, and can be bivalent or tetravalent.

Antibodies which specifically bind to polypeptide markers also can be produced by inducing in vivo production in the lymphocyte population or by screening immunoglobulin libraries or panels of highly specific binding reagents as disclosed in the literature.

Antibodies according to the invention can be purified by methods well known in the art. For example, antibodies can be affinity purified by passage over a column to which a polypeptide marker is bound. The bound antibodies can then be eluted from the column using a buffer with a high salt concentration.

A wide variety of labels and conjugation techniques are known by those skilled in the art and can be used in various nucleic acid and amino acid assays. Means for producing labelled hybridization or PCR probes for detecting sequences related to marker polynucleotides include oligo labelling, nick translation, end-labelling, or PCR amplification using a labelled nucleotide.

In the present invention, the terms "up-regulated" and "over-expressed" have the same meaning, can be interchangeably used and it has to be understood as that the marker is expressed over a reference value.

In the present invention, the terms "down-regulated" and "infra-expressed" have the same meaning, can be interchangeably used and it has to be understood as that the marker is expressed below a reference value.

In the present invention, the term "reference value" (also referred as "standard" or "reference") is to be understood as a predefined value of a given marker which is derived from the levels of said molecular marker in a sample or group of samples (hereinafter also referred as "reference samples"). If the level of expression is determined at the protein level, then the "reference value" is a predefined value of protein quantity, whereas if the level of expression is determined at the nucleic acid level, then the "reference value" is a predefined value of nucleic acid quantity. The samples are taken from a subject or group of subjects wherein the presence, absence, stage, or course of the disease has been properly performed previously. The samples, for instance, can be those already/previously classified in the clusters identified in the context of the invention. This value is used as a threshold to discriminate subjects wherein the condition to be analyzed is present from those wherein such condition is absent, to determine the stage of the disease and the aggressiveness of the cancer. This reference value is also useful for determining whether the subject will positively respond to therapy. The subject or subjects from whom the "reference value" is derived may include subject/s wherein the condition is absent, subject/s wherein the condition is present, or both.

Alternatively, the reference value is obtained from determining in the test sample the expression level of one or more housekeeping genes, which are constitutively expressed. Illustrative non-limitative examples of suitable genes for normalization include housekeeping genes such as ZNF346, ZKSCAN5, ZC3H14, TMUB2, SF3A3, GUSB, TUBB, and PPIA, using commercially available means. This normalization allows the comparison of the expression level in one sample, e.g., a subject sample, to another sample, e.g., a healthy subject, or between samples from different sources.

Software can be used to extract, normalize, summarize, and/or analyze the data by comparing against a reference set to determine whether differential expression is occurring in a sample. An increase or decrease can be indicative of an increase or decrease respectively of expression of the corresponding expressed sequence. An increase or decrease in relative intensity can also be indicative of a mutation in the expressed sequence.

The resulting values for each sample can be analyzed using feature selection techniques including filter techniques, which can assess the relevance of features by looking at the intrinsic properties of the data; wrapper methods, which embed the model hypothesis within a feature subset search; and/or embedded techniques in which the search for an optimal set of features is built into a classifier algorithm.

Filter techniques useful in the methods of the present invention can include (1) parametric methods such as the use of two sample t-tests, ANOVA analyses, Bayesian frameworks, and Gamma distribution models; (2) model free methods such as the use of Wilcoxon rank sum tests, between-within class sum of squares tests, rank products methods, random permutation methods, and/or TNoM (Threshold Number of Misclassifications) which involves setting a threshold point for fold-change differences in expression between two datasets and then detecting the threshold point in each gene that minimizes the number of misclassifications; (3) and multivariate methods such as bivariate methods, correlation based feature selection methods (CFS), minimum redundancy maximum relevance methods (MRMR), Markov blanket filter methods, and/or uncorrelated shrunken centroid methods. Wrapper methods useful in the methods of the present invention can include sequential search methods, genetic algorithms, and/or estimation of distribution algorithms. Embedded methods useful in the methods of the present invention can include random forest algorithms, weight vector of support vector machine algorithms, and/or weights of logistic regression algorithms.

Selected features can be classified using a classifier algorithm. Illustrative algorithms can include, but are not limited to, methods that reduce the number of variables such as principal component analysis algorithms, partial least squares methods, and/or independent component analysis algorithms. Illustrative algorithms can further include, but are not limited to, methods that handle large numbers of variables directly such as statistical methods and methods based on machine learning techniques. Statistical methods can include penalized logistic regression, prediction analysis of microarrays (PAM), methods based on shrunken centroids, support vector machine analysis, and regularized linear discriminant analysis. Machine learning techniques can include bagging procedures, boosting procedures, random forest algorithms, and/or combinations thereof.

The markers and genes of the present invention can be utilized to identify, classify, and/or characterize cells or tissues (e.g., as cancerous or benign, as from a male or female, as comprising a genetic mutation or wild-type, etc.).

The present invention includes methods for identifying, classifying, and/or characterizing tissues or cells comprising determining the differential expression of one or more markers or genes in a biological sample subject.

In one embodiment, optionally in combination with any of the embodiments provided above or below, the comparison of the expression level of the markers with a reference expression level is conducted using computer systems. In one embodiment, expression levels are obtained in two sets and these two sets of expression levels are introduced into a computer system for comparison. In another embodiment, one set of expression levels is entered into a computer system for comparison with values that are already present in the computer system, or in computer-readable form that is then entered into the computer system.

In one embodiment of the methods of the invention, optionally in combination with any of the embodiments provided above or below, step (b) is performed by comparing the expression level of each one of the markers with the expression level of the markers in one or more reference samples to determine an increase or decrease in expression level of said markers between the cancerous biological sample and the one or more reference samples.

In another embodiment of the method of the first aspect of the invention, optionally in combination with any of the embodiments provided above or below, the expression level of the markers in the cancerous biological sample is compared with: a percentile expression level, or with the mean expression level of said one or more markers the one or more reference samples, or both.

In an alternative embodiment of the methods of the invention, step (b) is performed by (i) determining the level of expression of each one of the markers in the test sample; and (ii) comparing the measured level with the level of one or more housekeeping genes.

As an alternative to make determinations based on the absolute expression level of the marker, determinations can be based on the normalized expression level of the marker.

The expression level can be provided as a relative expression level. To determine a relative expression level of a marker, the level of expression of the marker can be determined for 10 or more samples of normal versus cancer isolates, or even 50 or more samples, prior to the determination of the expression level for the sample in question. The mean expression level of each of the genes assayed in the larger number of samples can be determined and this can be used as a baseline expression level for the marker. The expression level of the marker determined for the test sample (absolute level of expression) then can be divided by the mean expression value obtained for that marker. This provides a relative expression level.

In certain embodiments, the samples used in the baseline determination will be from samples derived from a subject having cancer (versus samples from a healthy subject of the same tissue type. The choice of the cell source is dependent on the use of the relative expression level. Using expression found in normal tissues as a mean expression score aids in validating whether the marker assayed is specific to the tissue from which the cell was derived (versus normal cells). In addition, as more data is accumulated, the mean expression value can be revised, providing improved relative expression values based on accumulated data. Expression data from normal cells provides a means for grading the severity of the cancer disease state.

In another embodiment of the methods of the invention, optionally in combination with any of the embodiments provided above or below, step (b) is performed by determining the relative expression level of each one of the markers, which comprises: (b.1) determining the level of expression of each one of the markers; and (b.2) calculating a score (S) with the measured level of expression, for each one of the markers, following the formula (I):

$$S= (L-M)/s.d. \ (I)$$

wherein

L= the log2 of the expression level measured in step (b.1) for the marker;

M= the log 2 of the mean of the expression of the marker in a population of reference samples; and

s.d.= standard deviation of the mean (M).

If a score "S" is calculated, then it is considered the marker to be over-expressed when its value is above 0; and to be down-regulated when its value is below 0.

In one embodiment of the methods of the invention, the determination of the level of expression of other gene signatures are encompassed. Illustrative non-limitative examples of genetic signatures which can be considered in combination with the signature of the invention are: GEP signatures (such as the one disclosed in Cristescu R. et al., 2018), PD-1, PD-L1, and PD-L2. The determination of the level of expression of these further markers can be determined using commercially available reagents and following any of the techniques already disclosed above. The interpretation of these further data in conjunction with the data already obtained with the signature of the invention can be performed using well-known methods.

The present invention provides methods for accurately predicting the more appropriate anti-immune checkpoint therapy either using a statistical algorithm and/or the direct empirical data, the latter being obtained/calculated as disclosed in any of the above embodiments.

Regarding the statistical algorithm suitable in the context of the invention, it can be a single learning statistical classifier system. For example, a single learning statistical classifier system can be used to classify a sample as a based upon a prediction or probability, and the presence or level of the biomarker. The use of a single learning statistical classifier system typically classifies the sample as, for example, a likely anti-immune checkpoint -therapy responder or processor sample with a sensitivity, specificity, positive predictive value, negative predictive value, and/or overall accuracy of at least about 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%.

Other suitable statistical algorithms are well known to those of skill in the art. For example, learning statistical Classifier systems include a machine learning algorithmic technique capable of adapting to complex data sets (e.g., panel of markers of interest) and making decisions based upon such data sets.

In some embodiments, a single learning statistical classifier system such as a classification tree (e.g., random forest) is used. In other embodiments, a combination of 2, 3, 4, 5, 6, 7, 8, 9, 10, or more learning statistical classifier systems are used, preferably in tandem. Examples of learning statistical classifier systems include, but are not limited to, those using inductive learning (e.g., decision/classification trees such as random forests, classification and regression trees (C&RT), boosted trees, etc.), Probably Approximately Correct. (PAC) learning, connectionist learning (e.g., neural networks (NN), artificial neural networks (ANN), neuro fuzzy networks (NEN), network structures, perceptions such as multi-layer perceptions, multi-layer feed-forward networks, applications of neural networks, Bayesian learning in belief networks, etc), reinforcement learning (e.g., passive learning in a known environment such as naive learning, adaptive dynamic learning, and temporal difference learning, passive learning in an unknown environment, active learning in an unknown environment, learning action-value functions, applications of reinforcement learning, etc.), and genetic algorithms and evolutionary programming. Other learning statistical classifier systems include support vector machines (e.g, Kernel methods), multivariate adaptive regression splines (MARS), 'Levertberg-Marquardt algorithms, Gauss-Newton algorithms, mixtures of Gaussians, gradient descent algorithms, and learning vector quantization (LVQ).

A "machine learning algorithm" can refer to a computational-based prediction methodology, also known to persons skilled in the art as a "classifier", employed for characterizing a gene expression profile. The signals corresponding to certain expression levels, which can be obtained by, e.g., microarray-based hybridization assays, can be subjected to the algorithm in order to classify the expression profile. Supervised learning can involve "training" a classifier to recognize the distinctions among classes and then "testing" the accuracy of the classifier on an independent test set. For new, unknown samples, the classifier can be used to predict the class in which the samples belong.

Biological samples can be classified using a trained algorithm. Trained algorithms of the include algorithms that have been developed using two or more reference sets of known categorization.

Training can comprise comparison of gene expression product levels in a first set of one or more tissue types to gene expression product levels in a second set of one or more tissue types, where the first set of tissue types includes at least one tissue type that is not in the second set. In some cases, either the entire algorithm or portions of the algorithm can be trained using comparisons of expression levels of biomarker panels within a classification panel against all other biomarker panels (or all other biomarker signatures) used in the algorithm.

Algorithms suitable for categorization of samples include but are not limited to k-nearest neighbour algorithms, support vector algorithms, naive Bayesian algorithms, neural network algorithms, hidden Markov model algorithms, genetic algorithms, or any combination thereof.

In some cases, trained algorithms can incorporate data other than gene expression or alternative splicing data such as, but not limited to, DNA polymorphism data, sequencing data, scoring or diagnosis by cytologists or pathologists or information about the medical history of the subject.

In certain embodiments, the method of the present invention further comprises sending the sample classification results to a clinician e.g., an oncologist.

A computer system can also store and manipulate data generated by the methods of the present invention which comprises a plurality of biomarker signal changes/profiles which can be used by a computer system in implementing the methods of this invention.

In certain embodiments, optionally in combination with any of the embodiments provided above or below, (i) a computer system receives biomarker expression data; (ii) stores the data; and (iii) compares the data to determine the state of informative biomarkers from cancerous tissue.

In other embodiments, optionally in combination with any of the embodiments provided above or below, a computer system (i) compares the determined expression biomarker level to a threshold value; and (ii) outputs an indication of whether said biomarker level is significantly modulated (e.g., above or below) a threshold value, or a phenotype based on said indication.

In certain embodiments, optionally in combination with any of the embodiments provided above or below, such computer systems are also considered part of the present invention. Numerous types of computer systems can be used to implement the analytic methods of this invention according to knowledge possessed by a skilled artisan in the bioinformatics and/or computer arts.

The methods of the invention can also be programmed or modelled in mathematical software packages that allow symbolic entry of equations and high-level specification of processing, including specific algorithms to be used, thereby freeing a user of the need to procedurally program indiyidtial equations and algorithms.

Such packages include, e.g.. Matiab from Mathworks (Natick, Nlass.), Alathematica from Wolfram Research (Champaign, Hi) or S-Plus from MathSoft (Seattle, Wash.).

In certain embodiments, optionally in combination with any of the embodiments provided above or below, the computer comprises a database for storage of biomarker data. Stored profiles can be accessed and used to perform comparisons of interest at a later point in time. For example, biomarker expression profiles of a sample derived from the non-cancerous tissue of a subject and/or profiles generated from population-based distributions of informative loci of interest in relevant populations of the same species can be stored and later compared to that of a sample derived from the cancerous tissue of the subject.

In addition to the exemplary program structures and computer systems described herein, other, alternative program structures and computer systems will be readily apparent to the skilled artisan.

In one embodiment, optionally in combination with any of the embodiments provided above or below, the invention provides a computer readable form of the expression profile data of the invention, or of values corresponding to the level of expression of at least one in a diseased cell. For example, the values can be mRNA expression levels obtained from experiments, e.g., microarray analysis. The values can also be mRNA levels normalised relative to a reference gene whose expression is constant in numerous cells under numerous conditions. In other embodiments, the values in the computer are ratios of, or differences between, normalized or non-normalized mRNA levels in different samples.

The marker expression profile data can be in the form of a table, such as an Excel table. The data can be alone, or it can be part of a larger database, e.g., comprising other expression profiles. For example, the expression profile data of the invention can be part of a public database. The computer readable form can be in a computer.

In one embodiment, the invention provides a system that comprises a means for receiving gene expression data for one or a plurality of markers; a means for comparing the gene expression data from each of said one or plurality of genes to a common reference frame; and a means for presenting the results of the comparison. This system may further comprise a means for clustering the data.

With the information collected following the method(s) of the invention, the physician can recommend the most appropriate ICI-based therapy, as explained above.

Illustrative non-limitative examples of inhibitors of glycosylation due to increased HBP (GFAT1): 6-diazo-5-oxo-l-norleucine [DON]

Illustrative non-limitative examples of TGF-b inhibitors is galinusertib;

Illustrative non-limitative examples of CD39 inhibitors are: TTX-030 and SFR617;

Illustrative non-limitative examples of IDO1-AHR inhibitors is BMS-986205;

Illustrative non-limitative examples of MCTs inhibitors is AZD3965;

Illustrative non-limitative examples of inhibitors of autophagy are: SB02024 or SAR405;

Illustrative non-limitative examples of inhibitors of citrate transport are 4-chloro-3-[(3-nitrophenyl) amino] sulfonyl benzoic acid (mitochondrial citrate transport protein inhibitor or CTPi) (ChemBridge Corporation #6652048) or 2-(benzylsulfanyl)-N-[(pyridin-2-yl) methyl] propanamide (plasma membrane citrate transporter inhibitor or PMCTi) (TimTec ID ST056138)

Illustrative non-limitative examples of amino-acid transporter inhibitors: SLC7A5 inhibitor

Illustrative non-limitative examples of CDK4 inhibitors are: abemaciclib, ribociclib, and Trilaciclib;

Illustrative non-limitative examples of 6GPD inhibitors is dehydroepiandosterone; and

doble blocking strategies (to minimize ROS production and intrinsic antioxidant mechanisms): 2-deoxy-glucosa inhibitor +auranofin.

In a second aspect, the present invention provides a method of prognosis a patient suffering from cancer.

As intended herein, the expression "prognosis" refers to the prognosis of progression of cancer in a patient wherein the occurrence of a cancer has already been diagnosed, of various events, including: (i) the chances of occurrence of metastasis; (ii) the chances of occurrence of loco-regional recurrence of cancer, including colorectal cancer; and (iii) the chances of occurrence of a long disease-free (DFS) and/or long overall survival (OS) times; i.e. a DFS time or an OFS time of 5 years or more following testing with the in vitro prognosis method according to the invention. In one embodiment of the second aspect of the invention, optionally in combination with any of the embodiments provided above or below, the method comprises determining the overall survival coefficient using any available software package, such as SPSS® (IBM).

All the embodiments provided above, relating to the determination of the level of expression, reference value/samples, methods for comparing both data sets, combination with other signatures, and samples, among others, already provided under the first aspect of the invention, are also embodiments of the method of the second aspect of the invention.

In one embodiment of the second aspect of the invention, optionally in combination with any of the embodiments provided above or below, it is further determined the level of expression of one or more further markers selected from: GEP, and PD-1. As it is shown below, it was found that those samples falling in cluster 1 and having a GEP low (below 66th percentile) had a poorest overall survival. Whereas samples falling in cluster 2 or 3, exhibiting GEP high (above 66th percentile), had the best overall survival.

In a third aspect the present invention provides a kit.

In one embodiment, the kit comprises a binding panel of binding molecules that specifically binds to the biomarkers provided in the present invention, a detection reagent, appropriate solutions and instructions on how to use the kit. Such kits can be prepared from available known materials to those skilled in the art.

A binding molecule used within such a kit may include, but is not limited to, nucleic acids, nucleotides, oligonucleotides, polynucleotides, amino acids, peptides, polypeptides, proteins, monoclonal and/or polyclonal antibodies, sugars, carbohydrates, fatty acids, lipids, steroids, hormones, or a combination thereof.

Each binding molecule should be distinguishable from every other binding molecule in a panel of binding molecules, yielding easily interpreted signal for each of the biomolecules detected by the kit.

In another embodiment of the kit of the invention, optionally in combination with any of the embodiments provided above or below, the binding molecules (means) are selected from the group consisting of:

- at least one primer pair specific to each one of the markers, or alternatively

- at least one probe, each probe having a sequence complementary to the sequence of the markers, or alternatively,

- at least one primer pair specific to each one of the marker and at least one probe having a sequence complementary to the sequence of the marker; or, alternatively,

- at least one or more antibodies which specifically binds to each one of the protein markers.

There are commercially available primers, probes, and antibodies for determining the level of expression of the markers provided by the Nanostring.

Alternatively, the skilled person can design their own primers, probes and antibodies, making use of the general knowledge as well as of routine techniques and available bioinformatics softwares.

In one embodiment, optionally in combination with any of the embodiments provided above or below, the kit comprises at least one pair of primers for the amplification of each one of the markers. The primer must hybridize to the sequence to be amplified.

PCR primers can be designed routinely by those having ordinary skill in the art using sequence information.

Hence, the reverse oligonucleotide primer of the primer pairs may contain an oligo dT stretch of nucleotides, preferably eleven nucleotides long, at its 5' end, which hybridizes to the poly(A) tail of mRNA or to the complement of a cDNA reverse transcribed from an mRNA poly(A) tail. Second, in order to increase the specificity of the reverse primer, the primer may contain one or more, preferably two, additional nucleotides at its 3' end. Because, statistically, only a subset of the mRNA derived sequences present in the sample of interest will hybridize to such primers, the additional nucleotides allow the primers to amplify only a subset of the mRNA derived sequences present in the sample of interest. This embodiment allows a more accurate and complete visualization and characterization of each of the bands representing amplified sequences.

The forward primer may contain a nucleotide sequence expected, statistically, to have the ability to hybridize to cDNA sequences derived from the tissues of interest. The nucleotide sequence may be an arbitrary one, and the length of the forward oligonucleotide primer may range from about 9 to about 13 nucleotides, with about 10 nucleotides being preferred. Arbitrary primer sequences cause the lengths of the amplified partial cDNAs produced to be variable, thus allowing different clones to be separated by using standard denaturing sequencing gel electrophoresis.

Typical primers are 18-28 nucleotides in length and are generally have 50% to 60% G+C composition. The entire primer may be complementary to the sequence it must hybridize to. If mRNA is used as a template, the primers must hybridize to mRNA sequences. If cDNA is used as a template, the primers must hybridize to cDNA sequences.

In another embodiment, optionally in combination with any of the embodiments provided above or below, the kit comprises at least one antibody or a functional fragment thereof, either monoclonal or polyclonal, which specifically binds to each one of the expression product from the markers. All the embodiments provided above related to the antibodies useful in the context of the methods of the invention, are also embodiments of the kit of the invention.

In another embodiment, optionally in combination with any of the embodiments provided above or below, the kit is an immunoassay selected from: enzyme-linked immunosorbent assay (ELISA), radioimmunoassay (RIA), and fluorescence activated cell sorting (FACS).

In another embodiment, the kit may optionally further comprise a reference means so that the genes quantified within a biological sample can be compared with said reference (also referred hereinafter as "standard") to determine if the test amount of a marker detected in a sample is indicative of an up-regulation. Likewise, a biological sample can be compared with said standard to determine if the test amount of a marker detected in said sample is indicative of a down-regulation. In another embodiment of the first aspect of the invention, optionally in combination with any of the embodiments provided above or below, the kit further comprises means for determining the level of expression of one or more housekeeping markers, as defined above. Thus, when deciding whether one of the markers is up- or down-regulated the clinician would compare the selected genes with the housekeeping genes using, for example, by geometric mean.

In one embodiment, optionally in combination with any of the embodiments provided above or below, the kit further comprises means or instructions for determining the level of expression of one or more of PD-1, PD-L1 and GEP signature (Cristescu R. et al., 2018).

In one embodiment, the kit is an array wherein polynucleotide probes are immobilized on a DNA chip in an organized array. Oligonucleotides can be bound to a solid support by a variety of processes, including lithography. In one embodiment, the polynucleotide probes are spotted onto a substrate in a two-dimensional matrix or array, samples of polynucleotides can be labelled and then hybridized to the probes. Double-stranded polynucleotides, comprising the labelled sample polynucleotides bound to probe polynucleotides, can be detected once the unbound portion of the sample is washed away.

The probe polynucleotides can be spotted on substrates including glass, nitrocellulose, etc. The probes can be bound to the substrate by either covalent bonds or by non-specific interactions, such as hydrophobic interactions.

The sample polynucleotides can be labelled using radioactive labels, fluorophores, chromophores, etc. The probes and primers useful in the context of the kit of the invention are those already defined and identified above.

The kit can further comprise an adsorbent on a biologically active surface, wherein the adsorbent is suitable for binding means for detecting the biomarkers provided by the invention, and one or more of: denaturation solution for the pre-treatment of a sample, a binding solution, one or more washing solution(s), and instructions for making a denaturation solution, binding solution, and washing solution(s).

In another embodiment, optionally in combination with any of the embodiments provided above or below, the kit comprises instructions on how to use it, nucleotides, a polymerization enzyme (e.g. Taq polymerase) and buffer solution(s).

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

**Examples**

I. METHODS

1.1. Patient cohort

Human Samples

The use of human samples was approved by the Clinical Ethics Research Committee at the Hospital Clinic of Barcelona under reference HCB-2013/8674 and HCB-2018/0633. This study includes a retrospective cohort of 75 patients diagnosed with metastatic colorectal cancer between February 2016 and February 2020 at the Hospital Clinic of Barcelona. Data collection of patients was censored in November 2020. This cohort was artificially enriched with mCRC patients with BRAF genotype and MSI. Patients signed an informed consent approving the ulterior use of their tumor specimens in the GEMCAD collection repository. The protocol was approved by the Ethics Committee of the Hospital Clinic of Barcelona.

Clinic-pathologic information of patients was prospectively collected from the registry database at the Hospital Clinic of Barcelona. Key eligibility criteria included: diagnosis of stage IV disease with histologically confirmed adenocarcinoma within the colon or rectum. Clinical parameters utilized to initially evaluate patient's performance included: Sex, age, Eastern Cooperative Oncology Group (ECOG) performance status, stage at diagnoses, site of the primary lesion (descending colon, sigmoid and rectum were considered left sided), primary surgical resection, number of invaded organs, metastatic organ type, lactate dehydrogenase (LDH), alkaline phosphatase (ALP), C-reactive protein (PCR) levels, leukocytes concentration in blood and CEA levels. Microsatellite instability was also recorded.

A second cohort of 4200 TCGA samples from 11 tumor types: Kidney Renal Clear Cell Carcinoma (KIRC), Skin Cutaneous Melanoma (SKCM), Lung Adenocarcinoma (LUAD), Bladder Urothelial Carcinoma (BLCA), Head-Neck Squamous Cell Carcinoma (HNSC), Stomach Adenocarcinoma (STAD), ovarian cancer (OV), glioblastoma multiforme (GBM), Breast Cancer (BRCA-TNB), Colon Adenocarcinoma (COAD), Pancreatic adenocarcinoma (PAAD) was used to validate the information provided by the 10-marker signature.

RNA extraction and NanoString gene expression profiling

The NanoString 10360 panel on the nCounter platform (NanoString Technologies) was used to interrogate gene expression on formalin-fixed paraffin-embedded (FFPE) tumor tissues, following the manufacturer's protocol. Briefly, a section of formalin-fixed paraffin-embedded tumor tissues were first examined by H-E staining to determine the tumor surface area and cellularity. Only samples with a minimum of 10% tumor cellularity were processed for RNA purification (Roche® High Pure FFPET RNA isolation kit). The number of 10 $\mu$m-thick sections for processing were a function of the tumor area determined for each sample: 4-19 mm$^2$, 6 sections; 20-99 mm$^2$, 3 sections; >100 mm$^2$, 1 section. When needed, macrodissection was performed to enrich tumor cells and minimize stromal components.

Isolated RNA was tested to ensure it met the required specifications for concentration ($\geq$12.5 ng/$\mu$L) and purity (OD 260/280 nm, 1.7-2.3) using a NanoDrop® Spectrophotometer. After excluding samples with suboptimal RNA integrity and content, the remaining samples were included in the nCounter® analysis.

The final set of data that passed quality control (n = 75) were analyzed on the nSolver 4.0 Advanced Analysis module using default settings to derive the differentially expressed genes.

IMMETCOLS and pro-immune signature analysis

Nanostring (IO360) data were mean-centered and scaled by subtracting the mean and dividing by the standard deviation of each gene across all samples. A set of 35 manually selected immune and metabolic genes (CCL2, LDHA, SLC16A1, GLUL, BCAT1, GLUD1, CD80, ZEB1, CD8A, IL10, TGFB1, FASLG, TWIST1, PDCD1, SLC2A1, GLS, PC, CD163, CCL5, IL17A, IL2, S TAT1, IL6, SNAI1, ZEB2, TGFB3, FAP, VEGFA, VEGFB, PDK1, PKM, LDHB, SLC1A5, GOT1, ENTPD1) was used to cluster the samples into 3 distinct clusters with K-Means Clustering. This software grouped the samples on the basis of the expression profile of the 10 markers.

Then, a one-way ANOVA analysis was used to identify the genes most representative of each cluster. Among those genes, a subset of 10 genes was selected as a signature to stratify samples into each one of the three clusters (TGFB1, ZEB1, FAP, ZEB2, GLUL, ENTPD1, GOT1, LDHA, TWIST1, GLS).

These genes were used as features to train a neural network to classify prospective samples into the three clusters. To this end, the "nnet" implementation of the R Caret Package was used. A 10-fold cross-validation repeated 100 times was performed on the training data to fit the two hyperparameters of the neural network (size and decay). The expression profile of each one of the 4200 samples was included in this trained neural network and classified in one of the three clusters.

Samples were considered to be GEP-High if the average expression of genes of the GEP signature was above the 66th percentile of the mean expression of GEP genes in all analyzed sample tumors within the same cohort (either the one of 75 or 4200 samples).

Samples were considered to be PD1-High or PDL1-High if the expression of such markers was above the 75th percentile in all analyzed tumors within the same cohort (either the one of 75 or 4200 samples).

Statistical analysis

Gene counts for individual patients in each tumor type were normalized using the variable stabilizing transformation (VST) function of the DESeq2 package for R, following manufacturer's instructions.

Expression of each gene in the 10 gene signature was mean centered and scaled in each tumor type and used as input of the CARET predict function to stratify samples into the clusters, using the parameters by default and following manufacturer's instructions.

ANOVA was used to compare gene expression and immune signatures between samples assigned to each cluster.

Post-hoc analyses to identify which two levels are different is performed with Fisher's least significant difference method (Fisher's LSD).

Log-rank analysis was performed to determine the statistical significance of Kaplan-Meier survival curves using SPSS® (IBM, Armonk, NY, United States) software. Two time-toevent points were considered for survival analysis: the primary endpoint was overall survival (OS), defined as the time from metastatic diagnosis to death from any causes (for deceased patients) or the last follow-up; and the secondary end point was progressionfree survival (PFS), defined from the date of metastatic diagnoses to the date of progression or death.

For univariate and multivariate analysis descriptive statistics were used to test their correlation with OS. For all statistical methods, $p < 0.05$ was considered significant.

II. RESULTS

A simplified immune-metabolic signature (IMMETCOLS) identify three different subgroups of cancer patients with distinct metabolic profiles

Data from 75 metastatic colorectal cancer cases were interrogated for expression of immune-metabolic genes. It was found that the 75 samples could be classified, using K-Means clustering, into three clusters just analysing 10 genes: TGFB1, ZEB1, FAP, ZEB2, GLUL, ENTPD1, GOT1, LDHA, TWIST1, and GLS.

Then, the one-way ANOVA was performed to confirmed how differentially expressed was each one of the 10 gene markers in each one of the clusters using Fisher least significant difference method. The results are summarized in Table 1 below:

Table1 Anova analysis of the expression of genes in each cluster. Fisher's least significant difference method indicates the genes significant between each cluster.

| | p.value | FDR | Fisher's LSD |
|---|---|---|---|
| ZEB1 | 3.70E-16 | 1.30E-14 | Cluster_1- Cluster_2; Cluster_1 - Cluster_3; Cluster_3 - Cluster_2 |
| ZEB2 | 9.36E-16 | 1.53E-14 | Cluster_1 - Cluster_2; Cluster_1 - Cluster 3; Cluster_3 - Cluster_2 |
| ENTPD1 | 1.31E-15 | 1.53E-14 | Cluster_1 - Cluster_2; Cluster_1 - Cluster_3; Cluster_3 - Cluster_2 |
| LDHA | 3.48E-15 | 3.04E-14 | Cluster_1 - Cluster_2; Cluster_3 - Cluster_2 |
| FAP | 5.95E-13 | 2.98E-12 | Cluster_1 - Cluster_2; Cluster_1 - Cluster_3 |
| GLS | 4.08E-12 | 1.78E-11 | Cluster_2 - Cluster_1 ; Cluster_2 - Cluster_3 |
| GOT1 | 2.96E-11 | 1.03E-10 | Cluster_1 - Cluster_2; Cluster_3 - Cluster_1; Cluster_3 - Cluster_2 |
| TGFB1 | 4.84E-11 | 1.41E-10 | Cluster_1 - Cluster_3; Cluster_2 - Cluster_3 |
| TWIST1 | 1.99E-08 | 3.88E-08 | Cluster_1 - Cluster_3; Cluster_2 - Cluster_3 |
| GLUL | 7.83E-07 | 1.31E-06 | Cluster_1 - Cluster_2; Cluster_1 - Cluster_3 |

Thus, the three clusters were defined as follows:

- Cluster 1: the following genes tend to be upregulated compared to a reference sample derived from other clusters: ZEB1, ZEB2, ENTPD1, FAP and GLUL;

- Cluster 2: GLS tend to be upregulated, and LDHA, GOT1, ZEB1 and ZEB2 and ENTPD1 to be downregulated compared to a reference sample derived from other clusters; and

- Cluster 3: GOT1 tends to be upregulated and TGFB1 and TWIST1 to be downregulated compared to a reference sample derived from other clusters.

The above results were further confirmed using a cohort of 4200 TCGA samples in 11 tumor types: Kidney Renal Clear Cell Carcinoma (KIRC), Skin Cutaneous Melanoma (SKCM), Lung Adenocarcinoma (LUAD), Bladder Urothelial Carcinoma (BLCA), Head-Neck Squamous Cell Carcinoma (HNSC), Stomach Adenocarcinoma (STAD), ovarian cancer (OV), glioblastoma multiforme (GBM), Breast Cancer (BRCA-TNB), Colon Adenocarcinoma (COAD), Pancreatic adenocarcinoma (PAAD).

These tumor types were selected as they have been already reported in the prior art as showing different pembrolizumab efficacy: those which are pembrolizumab sensitive, if BOR was between 28-36% (SKCM, LUAD, KIRC and BLCA), those moderately sensitive, if BOR was between (7-17%) (BRCA-TNB, OV, HNSC, STAD), and pembrolizumab resistant, if BOR was between 0-8% (COAD-MSS, PAAD, GBM) using the predict method of the Caret package.

First of all, it was surprisingly found that just using just 10 markers, a precise information of the metabolic/immunologic nature of each one of the 4200 cancer samples: all of them could be classified in one of the three clusters. Not only that, but also, thanks to the information provided, when the sample is classified in one or other cluster, the physician has a valuable information about the essence and nature of the cancer, being possible the definition of a more accurate immune-based therapy.

Once it was confirmed the samples falling in each one of the clusters, the inventors performed a deep characterization from the point of view of metabolomics to confirm the robustness of the signature. The inventors found that there was a correlation between the information already provided by the clusters 1 to 3 and the affected metabolic pathways. Concluding that distinct metabolic characteristics with significant intensity differences in key enzymes of main carbon metabolism pathways were found.

When this exhaustive characterization of the samples was performed, the following was found:

Cluster 1 was enriched in cancer associated fibroblasts (CAFs) and EMT markers. This cluster showed high immune-suppression due to the presence of Tregs, MDSC and also exhausted CD8+ T cells. Metabolic reprogramming was defined by a Warburg metabolism with up-regulated glucose transporters GLUT1 (SLC2A1), and GLUT3 (SLC2A3), lactate transporters transporters MCT1 (SLC16A1) and MCT4 (SLC16A3), and glycolytic enzymes hexokinase 1 (HK1), PGM1, PGM2, 6-phosphofructo-2-kinase/fructose-2,6-biphosphatase-3 (PFKFB3) and lactate dehydrogenase A (LDHA). Cluster 1 displayed also enhanced gene expression of key enzymes in the hexosamine biosynthesis pathway (HBP) such as GFAT2, that culminates in the synthesis of uridine diphosphate N-acetylglucosamine (UDP-GlcNAc), an amino sugar essential for glycosylation and hyaluronan synthase 1 (HAS1) that converted UDP-GlcNAc into HA, a key glycancomponent of extracellular tumor matrix. Cluster 1 was also characterized by unique overexpression of three glutamine transporters (SLC38A1, SLC38A2, SLC38A5) and BCAT1, the cytosolic branched-chain-amino-acid aminotransferase isoform. Thus, glutamate produced as a by-product of enhanced HBP and lactate in cancer cells, could sustain BCAA synthesis through BCKA secreted by CAFs which are over-represented also in cluster 1. Macropinocytosis (ARHGEF2 and CaMKK2) was also up-regulated in cluster 1. Consistent with the up-regulation of immune infiltrates biomarkers in cluster 1, there is also an up-regulation of IDO (Indoleamine 2,3-dioxygenase 1) SLC7A7 transporters, the rate-limiting step in the conversion of tryptophan into the immunosuppressant metabolite kynurenine (Kyn) and CD39 which are all upregulated in cluster 1.

Cluster 2 was characterized to be an upregulation of SLC38a3, which is the glutamine transporter predominantly expressed in gluconeogenic tissues such as liver and kidney, glutaminase (GLS1 and GLS2), and glutamate dehydrogenase (GLUD2). The upregulation of this pathway results in the conversion of glutamine into $\alpha$-ketoglutarate ($\alpha$KG) that serves as substrate for gluconeogenesis. This cluster was also characterized by the over-expression of SLC2A12 (urate transporter) and glutamine synthase (GLUL), an enzyme that has been reported to be over-expressed in adaptation to glutamine deprived tumor microenvironment. Consistently, our results show that the gene expression of glycolysis transporters and key glycolytic enzymes expression is lower in cluster 2 which is indicative that these tumors rely on other carbon sources to sustain their growth needs. By contrary, cluster 2 specifically displays upregulation of aldolase B (ALDB), that is the isoform mainly expressed in gluconeogenic tissues, and of both fructose-1,6-bisphosphatase isoenzymes (FBP1 and FBP2), which are the key enzymes in gluconeogenesis pathway and are also involved in regulation of glycerol synthesis. Phosphoenolpyruvate carboxykinase (PCK2), the other key enzyme controlling gluconeogenesis, is also upregulated in cluster 2; even this feature was also observed in cluster 3. Up-regulation of gluconeogenesis is in accordance with up-regulation of SLC5A12 and SLC5A8 which are Na+ dependent transporters that import short-chain fatty acids, ketone bodies and other gluconeogenic precursors into the cells.

Acetyl-CoA synthetase (ACSS2) and Glycerol-3-phosphate dehydrogenase (GPD1) were also up-regulated in cluster 2 as well as glycerol kinase (GK) which was indicative of an active lipid metabolism and lipid uptake. In accordance, this cluster was also enriched on long-chain fatty acid (LCFA) transporter and SLC27A6 (FATP6) that are involved in the translocation of LCFA across the plasma membrane, and plasma membrane citrate transporters (SLC13A2 and SLC13A5), that facilitate citrate import supporting fatty acid synthesis under nutrient-limited conditions. Key enzymes in BCAA catabolism (BCAT2 and BCKDHA) are also up-regulated in this cluster which is indicative that BCAA are also used as a fuel for mitochondrial ATP production. Finally, cluster 2 is particularly enriched in V-ATPase, critical genes implicated in K+, Na+ and Cl- transport (CLCNKB, SLC12A1 and KCNJ1) and the canonical autophagy pathway (FIP200, ULK1, ATG13, ATG14 and NBR1).

Cluster 3 was characterized by the up-regulation of GLUT1 (SLC2A1), glycolytic enzymes (hexokinase 2 (HK2), phosphofructokinase (PFKI and PFKP), pyruvate kinase 1 (PKM1), PFKFB4, glycogen synthase isoenzymes (GYS1 and GYS2) and key players in pentose phosphate cycle (glucose-6-phosphate dehydrogenase (G6PD) and transketolase (TK) that supports the synthesis of NADPH. BCAA catabolism pathway (SLC3A2, BCA2 and BCKDK) as well as key players in conferring capacity to perform reductive carboxylation, from a-ketoglutarate to citrate, such as pyruvate carboxylase (PC), isocitrate dehydrogenase (IDH2), and key enzymes in proline biosynthesis, and one carbon and folate metabolism are also up-regulated. Additional specific characteristic was the upregulation of key hypoxia-induced transporters related to amino acid cellular import SLC1A5 and SLC7A5 (a glutamine antiporters). Also OXPHOS (key players in Complex I-IV), several mitochondrial carriers of SLC25 family, that have been reported to be directly or indirectly involved in inflammation are overexpressed: SLC25A22 (mitochondrial, import glutamate and H+), SLC25A1 (mitochondrial antiporter that export citrate in exchange with malate), SLC25A18 (transport of glutamate across inner membrane together with H+) and the two receptors mainly involved in malate-aspartate shuttle SLC25A13 (antiport that import glutamate and export aspartate) and SLC25A11 (antiport that can transports 2-ketoglutarate, malate and also succinate). In accordance with the up-regulation of malate-aspartate shuttle it has been also observed an upregulation of aspartate aminotransferases (GOT1). Upregulation of several cell cycle genes is also a characteristic of this cluster.

_Relevance of the signature of the invention to predict candidate therapeutic regimens based on the administration of check-inhibitors_

According to the Cluster 1 immunometabolic phenotype, the candidate therapeutic approach is based on enhancing ICI efficacy by combination with GFPT2 inhibitors through depleting glycan enriched tumor microenvironment. Additional putative combined therapies to be explored could be immune-checkpoint blockade combined with either TGFb inhibitors, CD39 inhibitors, IDO1-AHR inhibitors or with MCTs (lactate transporters) inhibitors.

According to the Cluster 2 immunometabolic phenotype, the candidate therapeutic approach is based on combining ICI with targeting citrate transporters to interfere with cancer cell uptake of lipogenesis precursors and lipid synthesis; as well as to uratelowering therapies already used in cancer treatment, to interfere with macrophage-cancer cells metabolic interdependence through urate. In addition, inhibition of lysosomal V-ATPase and autophagy could also restore MCH levels and immune sensitivity in combination with ICI.

According to cluster 3 immunometabolic phenotype, the candidate therapeutic approach is based on combining ICI with glucose transporter inhibitors, amino-acid transporter inhibitors, CDK4 inhibitors, one-carbon-folate and/or 6GPD inhibitors or doble blocking strategies (to minimize ROS production and intrinsic antioxidant mechanisms of defense).

_Association of the signature of the invention with pro-immune signatures_

To explore the utility of the signature of the invention to classify across tumor types, the relationship among the signature of the invention and proinflammatory signatures (Immune infiltration signatures (T-cell inflamed gene expression signature (GEP), CD274 (PD-L1), PD-L2, PD-1 and immunophenoscore) was further explored in TCGA (n=4200 patients).

Patients were stratified by GEP (below the top tertile data), PD-1, and PD-L1 (below the top quartile data). The GEP signature (FDR=1.91E-08), PD-L1 gene expression (FDR=5.16E-06), and PD-1 expression (FDR=0.00092041) were found to be overexpressed in Cluster 1 (data not shown). By contrast high Immunophenoscore signature (which measure 4 different parameters such as MHC expression, effector cells (EC), checkpoint immunomodulators (CP) and suppressor cells (SC)) was higher in Cluster 2 and 3 (FDR=0.0002118). It's worthy to emphasize that Cluster 1 despite showing the highest MHC, had the lower immunophenoscore due to the high presence of CP and SC. Finally, it was found that tumors refractory to pembrolizumab therapy (COAD-MSS, PAAD, GBM) with high GEP or PD-1 expression, fulfil almost entirely in the immunosuppressive cluster 1.

_Relevance of IMMETCOLS signature in patient prognosis_

It was firstly assessed the prognostic value of each signature (GEP, PD-1 and IMMETCOLS) across 11 tumors. Patients with high GEP and high PD-1 had longer overall survival, however I those samples falling in cluster 1 exhibited poor survival in the univariate analysis (Fig 3A and 3B). Next, it was considered whether the combination of GEP and IMMETCOLS distinguish diverse prognosis. Patients with high GEP and cluster 2 or 3 had the best OS and low GEP and IMMETCOLS cluster 1 had the poorest OS.

The above results further allow to conclude that the 10-marker signature of the invention, in conjunction with GEP signature can increase survival prediction in the multivariate analysis.

**Citation List**

Burtis C. A. et al., 2008, Chapter 14, section "Statistical Treatment of Reference Values";

Cristescu R. et al., "Pan-tumor genomic biomarkers for PD-1 checkpoint blockade-based immunotherapy", Science, 2018, vol. 362, page 3593; DOI: 10.1126/science.aar3593

Charoentong P. et al., "Pan-cancer Immunogenomic Analyses Reveal Genotype-Immunophenotype Relationships and Predictors of Response to Checkpoint Blockade", Cell Rep. 2017 Jan 3;v. 18(1), p. 248-262;

# EP 4 137 585 A1

Marabelle A. et al., "Association of tumour mutational burden with outcomes in patients with advanced solid tumours treated with pembrolizumab: prospective biomarker analysis of the multicohort, open-label, phase 2 KEYNOTE-158 study", Lancet Oncol., 2020, vol. 21(10), p. 1353-1365;

Pare L. et al., "Association between PD1 mRNA and response to anti-PD1 monotherapy across multiple cancer types", Ann Oncol., 2018, vol. 29(10), p. 2121-2128; and

Shitara K et al., "Efficacy and Safety of Pembrolizumab or Pembrolizumab Plus Chemotherapy vs Chemotherapy Alone for Patients With First-line, Advanced Gastric Cancer: The KEYNOTE-062 Phase 3 Randomized Clinical Trial. JAMA Oncol. 2020 Oct 1;6(10):1571-1580.

**Clauses**

For reasons of completeness, various aspects of the invention are set out in the following numbered clauses:

**Clause 1.** A method for deciding a check-point inhibitor (ICI)-based therapy, for a patient suffering from cancer, the method comprising the steps:

> a) measuring the expression level of the following markers: TGFB1, ZEB1, FAP, ZEB2, GLUL, ENTPD1, GOT1, LDHA, TWIST1, and GLS, in an isolated cancerous biological sample from the patient;
>
> b) determining which markers are differentially expressed; and
>
> c) classifying the sample, depending on the markers differentially expressed, in one of the following clusters:
>
>> cluster 1: when ZEB1, ZEB2, ENTPD1, FAP and GLUL are up-regulated;
>>
>> cluster 2: when GLS is upregulated; and LDHA, GOT1, ZEB1, ZEB2 and ENTPD1 are downregulated; and
>>
>> cluster 3: when GOT1 is upregulated and TGFB1 and TWIST1 are downregulated; wherein:
>>
>>> when the sample is classified in "cluster 1" then it is indicative that the patient is candidate to benefit from a combined therapy comprising a check-point inhibitor (ICI) therapy and one or more of: TGF-b inhibitors, CD39 inhibitors, IDO1-AHR inhibitors and/or MCTs inhibitors;
>>>
>>> when the sample is classified in "cluster 2", then it is indicative that the patient is candidate to benefit from a combined therapy comprising ICI therapy and one or more of: drugs targeting citrate transporters and an inhibitor of the lysosomal V-ATPase and autophagy; and
>>>
>>> when the sample is classified in "cluster 3", then it is indicative that the patient is candidate to benefit from a combined therapy comprising ICI therapy with one or more of: glucose transporter inhibitors, amino-acid transporter inhibitors, CDK4 inhibitors, one-carbon-folate, and/or 6GPD inhibitors; and doble blocking strategies (to minimize ROS production and intrinsic antioxidant mechanisms).

**Clause 2**. A method for deciding a check-point inhibitor (ICI)-based therapy, for a patient suffering from cancer, the method comprising the steps:

> a) measuring the expression level of the following markers: TGFB1, ZEB1, FAP, ZEB2, GLUL, ENTPD1, GOT1, LDHA, TWIST1, and GLS, in an isolated cancerous biological sample from the patient;
>
> b) determining which markers are differentially expressed; and
>
> c) classifying the sample, depending on the markers differentially expressed, in one of the following clusters:
>
>> cluster 1: when ZEB1, ZEB2, ENTPD1, FAP and GLUL are up-regulated;
>>
>> cluster 2: when GLS is upregulated; and LDHA, GOT1, ZEB1, ZEB2 and ENTPD1 are downregulated; and

cluster 3: when GOT1 is upregulated and TGFB1 and TWIST1 are downregulated; wherein:

when the sample is classified in "cluster 1" then it is indicative that the patient is candidate to benefit from a combined therapy comprising a check-point inhibitor (ICI) therapy and one or more of: GFPT2 inhibitors,-TGF-b inhibitors, CD39 inhibitors, IDO1-AHR inhibitors and/or MCTs inhibitors;

when the sample is classified in "cluster 2", then it is indicative that the patient is candidate to benefit from a combined therapy comprising ICI therapy and one or more of inhibitors of autophagy; and

when the sample is classified in "cluster 3", then it is indicative that the patient is candidate to benefit from a combined therapy comprising ICI therapy with one or more of: amino-acid transporter inhibitors, CDK4 inhibitors, and/or 6GPD inhibitors.

**Clause 3.** The method of any one of the preceding clauses, wherein step (b) is performed by comparing the expression level of each one of the above markers with the expression level of:

- the same markers in one or more reference samples; or, alternatively,

- with one or more housekeeping genes;

to determine an increase or decrease in expression level of said markers between the cancerous biological sample and the one or more reference samples.

**Clause 4.** The method of any one of the preceding clauses, wherein the expression level of the markers in the cancerous biological sample is compared with: a percentile expression level of these same markers in the one or more reference samples, or with the mean expression level of said one or more markers in the one or more reference samples, or both.

**Clause 5.** The method of any one of the preceding clauses, wherein step (b) is performed by

b.1. determining the level of expression of each one of the markers; and

b.2. normalizing the measured level, such as calculating a score (S), for each one of the markers, following the formula (I):

$$S= (L-M)/s.d. \quad (I)$$

wherein

L= the log2 of the expression level measured in step (b.1) for the marker;

M= the log 2 of the mean of the expression of the marker in a population of reference samples; and

s.d.= standard deviation of the mean (M).

**Clause 6.** The method of the preceding clause, wherein the marker is considered to be over-expressed when the S value is above 0; and to be down-regulated when the S value is below 0.

**Clause 7.** The method of any one of the preceding clauses, wherein the marker is a nucleic acid molecule or a peptide.

**Clause 8**. The method of any one of the preceding clauses, wherein the marker is a nucleic acid molecule, particularly RNA.

**Clause 9.** The method of any of the above clauses, wherein the checkpoint inhibitor inhibits a checkpoint protein selected from the group consisting of: cytotoxic T-lymphocyte antigen-4 (CTLA4), programmed cell death protein 1 (PD-1), PD-L1, PD-L2, B7-H3, B7-H4, herpesvirus entry mediator (HVEM), T cell membrane protein 3 (TIM3), galectin 9 (GAL9), lymphocyte activation gene 3 (LAG3), V-domain immunoglobulin (Ig)-containing suppressor of T-cell activation (VISTA), Killer-Cell Immunoglobulin-Like Receptor (KIR), B and T lymphocyte attenuator (BTLA), T cell immunoreceptor with Ig and ITIM domains (TIGIT), and combinations thereof.

**Clause 10.** The method of the preceding clause, wherein the checkpoint inhibitor inhibits CTLA-4, PD-1 or PD-L1.

**Clause 11.** The method of any one of the preceding two clauses, wherein the checkpoint inhibitor inhibits CTLA-4 and is selected from Ipilimumab and Tremelimumab.

**Clause 12.** The method of any one of the clauses 10-11, wherein the checkpoint inhibitor inhibits PD-1 and is selected from Nivolumab, Pembrolizumab, Pidilizumab, lambrolizumab, and AMP-224.

Clause 13. The method of any one of the clauses 10-11, wherein the checkpoint inhibitor inhibits PD-L1 and is selected from BMS-936559, MEDI-4736, MPDL33280A, M1H1, Atezolizumab, Durvalumab and Avelumab.

Clause 14. The method of any one of the preceding clauses, wherein:

the TGF-b inhibitors is galinusertib;

the CD39 inhibitor are selected from: TTX-030 and SFR617;

the IDO1-AHR inhibitor is selected from: BMS-986205;

the MCTs inhibitor is AZD3965;

the inhibitors of autophagy are selected from: SB02024 and SAR405;

the amino-acid transporter inhibitor is SLC7A5 inhibitor;

the CDK4 inhibitor is selected from: abemaciclib, ribociclib, Trilaciclib; and

the 6GPD inhibitor is dehydroepiandosterone.

**Clause 15.** A method of determining the prognosis of a patient suffering from cancer:

a) measuring the expression level of the markers TGFB1, ZEB1, FAP, ZEB2, GLUL, ENTPD1, GOT1, LDHA, TWIST1, and GLS in a cancerous biological sample obtained from the patient; and

b) determining which markers are differentially expressed; and

c) classifying the sample, depending on the markers differentially expressed, in one of the following clusters:

cluster 1: when ZEB1, ZEB2, ENTPD1, FAP and GLUL are up-regulated;

cluster 2: when GLS is upregulated; and LDHA, GOT1, ZEB1, ZEB2 and ENTPD1 are downregulated; and

cluster 3: when GOT1 is upregulated and TGFB1 and TWIST1 are downregulated; wherein:
when the sample is classified in "cluster 1" then it is indicative that the patient has a bad prognosis; whereas when the sample is classified in any of the clusters 2 or 3, the patient has a better prognosis.

**Clause 16.** The method of clause 15, wherein step (b) is performed by comparing the expression level of each one of the above markers with the expression level of the markers in one or more reference samples to determine an increase or decrease in expression level of said markers between the cancerous biological sample and the one or more reference samples.

**Clause 17.** The method of any one of the clauses 15-16, wherein the expression level of the markers in the cancerous biological sample is compared with: a percentile expression level of these same markers in the one or more reference samples, or with the mean expression level of said one or more markers in the one or more reference samples, or both.

**Clause 18.** The method of clause 17, wherein step (b) is performed by

b.1. determining the level of expression of each one of the markers; and
b.2. normalizing the measured level, such as calculating a score (S) with the measured level of expression, for each one of the markers, following the formula (I):

$$S= (L-M)/s.d. \ (I)$$

wherein

L= the log2 of the expression level measured in step (b.1) for the marker;

M= the log 2 of the mean of the expression of the marker in a population of reference samples; and

s.d.= standard deviation of the mean (M).

**Clause 19.** The method of the preceding clause, wherein the marker is considered to be over-expressed when the S value is above 0; and to be down-regulated when the S value is below 0.

**Clause 20.** The method of any one of the preceding clauses, wherein the marker is a nucleic acid molecule or a peptide.

**Clause 21.** The method of any one of the preceding clauses, wherein the marker is a nucleic acid molecule, particularly RNA.

**Clause 22.** The method of any of the previous clauses, wherein the biological sample is selected from tissue or a fluid sample such as blood, plasma, or serum, particularly a tissue, particularly a paraffin-embedded tissue.

**Clause 23.** The method of any of the previous clauses, wherein the cancer is selected from Kidney Renal Clear Cell Carcinoma (KIRC), Skin Cutaneous Melanoma (SKCM), Lung Adenocarcinoma (LUAD), Bladder Urothelial Carcinoma (BLCA), Head-Neck Squamous Cell Carcinoma (HNSC), Stomach Adenocarcinoma (STAD), ovarian cancer (OV), glioblastoma multiforme (GBM), Breast Cancer (BRCA-TNB), Colon Adenocarcinoma (COAD), Pancreatic adenocarcinoma (PAAD), and Colorectal Carcinoma (CRC).

**Clause 24.** A kit comprising means for determining the expression of the markers TGFB1, ZEB1, FAP, ZEB2, GLUL, ENTPD1, GOT1, LDHA, TWIST1, and GLS.

**Clause 25.** The kit of clause 24, wherein the means are selected from the group consisting of:

- at least one primer pair specific to each one of the markers, or alternatively
- at least one probe, each probe having a sequence complementary to the sequence of the markers, or alternatively,
- at least one primer pair specific to each one of the markers and at least one probe having a sequence complementary to the sequence of the markers, or, alternatively,
- at least one or more antibodies which specifically binds to each one of the markers.

**Clause 26.** The kit of any one of the clauses 24-25, which is an array.

**Clause 27.** Use of a kit as defined in any one of the clauses 24-26 in a method as defined in any of the above clauses 1-23.

**Claims**

1. A method for deciding a check-point inhibitor (ICI)-based therapy, for a patient suffering from cancer, the method comprising the steps:

a) measuring the expression level of the following markers: TGFB1, ZEB1, FAP, ZEB2, GLUL, ENTPD1, GOT1, LDHA, TWIST1, and GLS, in an isolated cancerous biological sample from the patient;
b) determining which markers are differentially expressed; and
c) classifying the sample, depending on the markers differentially expressed, in one of the following clusters:

cluster 1: when ZEB1, ZEB2, ENTPD1, FAP and GLUL are up-regulated;
cluster 2: when GLS is upregulated; and LDHA, GOT1, ZEB1, ZEB2 and ENTPD1 are downregulated; and
cluster 3: when GOT1 is upregulated and TGFB1 and TWIST1 are downregulated; wherein:

when the sample is classified in "cluster 1" then it is indicative that the patient is candidate to benefit from a combined therapy comprising a check-point inhibitor (ICI) therapy and one or more of: GFPT2 inhibitors,-TGF-b inhibitors, CD39 inhibitors, IDO1-AHR inhibitors and/or MCTs inhibitors;
when the sample is classified in "cluster 2", then it is indicative that the patient is candidate to benefit from a combined therapy comprising ICI therapy and one or more of: drugs targeting citrate transporters and an inhibitor of the lysosomal V-ATPase and autophagy; and
when the sample is classified in "cluster 3", then it is indicative that the patient is candidate to benefit from a combined therapy comprising ICI therapy with one or more of: glucose transporter inhibitors, amino-acid transporter inhibitors, CDK4 inhibitors, one-carbon-folate, and/or 6GPD inhibitors.

2. The method of claim 1, wherein step (b) is performed by comparing the expression level of each one of the above markers with the expression level of:

- the same markers in one or more reference samples; or, alternatively,
- with one or more housekeeping genes;

to determine an increase or decrease in expression level of said markers between the cancerous biological sample and the one or more reference samples.

3. The method of claim 2, wherein step (b) is performed by

b.1. determining the level of expression of each one of the markers; and
b.2. normalizing the measured, such as calculating a score (S) with the following the formula (I):

$$S = (L-M)/s.d. \quad (I)$$

wherein

L= the log2 of the expression level measured in step (b.1) for the marker;
M= the log 2 of the mean of the expression of the marker in a population of reference samples; and
s.d.= standard deviation of the mean (M).

4. The method of claim 3, wherein the marker is considered to be over-expressed when the S value is above 0; and to be down-regulated when the S value is below 0.

5. The method of any one of the preceding claims, wherein the marker is a nucleic acid molecule or a peptide.

6. The method of any one of the preceding claims, wherein the marker is a nucleic acid molecule, particularly RNA.

7. The method of any of the above claims, wherein the checkpoint inhibitor inhibits a checkpoint protein selected from the group consisting of: cytotoxic T-lymphocyte antigen-4 (CTLA4), programmed cell death protein 1 (PD-1), PD-LI, PD-L2, B7-H3, B7-H4, herpesvirus entry mediator (HVEM), T cell membrane protein 3 (TIM3), galectin 9 (GAL9), lymphocyte activation gene 3 (LAG3), V-domain immunoglobulin (Ig)-containing suppressor of T-cell activation (VISTA), Killer-Cell Immunoglobulin-Like Receptor (KIR), B and T lymphocyte attenuator (BTLA), T cell immunoreceptor with Ig and ITIM domains (TIGIT), and combinations thereof.

8. The method of any of the preceding claims, wherein the biological sample is selected from tissue or a fluid sample such as blood, plasma, serum, particularly a tissue, particularly a paraffin-embedded tissue.

9. The method of any of the preceding claims, wherein the cancer is selected from Kidney Renal Clear Cell Carcinoma (KIRC), Skin Cutaneous Melanoma (SKCM), Lung Adenocarcinoma (LUAD), Bladder Urothelial Carcinoma (BLCA), Head-Neck Squamous Cell Carcinoma (HNSC), Stomach Adenocarcinoma (STAD), ovarian cancer (OV), glioblastoma multiforme (GBM), Breast Cancer (BRCA-TNB), Colon Adenocarcinoma (COAD), Pancreatic adenocarcinoma (PAAD), and Colorectal Carcinoma (CRC).

10. A kit comprising means for determining the expression of the markers TGFB1, ZEB1, FAP, ZEB2, GLUL, ENTPD1, GOT1, LDHA, TWIST1, and GLS.

11. The kit of claim 10, wherein the means are selected from the group consisting of:

- at least one primer pair specific to each one of the markers, or alternatively
- at least one probe, each probe having a sequence complementary to the sequence of the markers, or alternatively,
- at least one primer pair specific to each one of the markers and at least one probe having a sequence complementary to the sequence of the markers, or, alternatively,
- at least one or more antibodies which specifically binds to each one of the markers.

12. The kit of any one of the claims 10-11, which is an array.

13. Use of a kit as defined in any one of the claims 10-12 in a method as defined in any of the above claims 1-9.

Europäisches
Patentamt

European
Patent Office

Office européen
des brevets

# EUROPEAN SEARCH REPORT

**Application Number**

EP 21 38 2772

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/090263 A1 (GENENTECH INC [US]; HOFFMANN LA ROCHE [CH]) 9 May 2019 (2019-05-09) | 10-12 | INV. C12Q1/6886 |
| A | * abstract; claim 1 * | 1-9,13 | |
| X | MARK AYERS ET AL: "IFN-γ–related mRNA profile predicts clinical response to PD-1 blockade", THE JOURNAL OF CLINICAL INVESTIGATION, vol. 127, no. 8, 1 August 2017 (2017-08-01), pages 2930-2940, XP055608325, GB ISSN: 0021-9738, DOI: 10.1172/JCI91190 | 10-12 | |
| A | * abstract; figures 5A, 5B * * page 2932, column 2 - page 2933, column 2 * | 1-9,13 | |
| X | LI YING ET AL: "A Novel Prognostic Signature Based on Metabolism-Related Genes to Predict Survival and Guide Personalized Treatment for Head and Neck Squamous Carcinoma", FRONTIERS IN ONCOLOGY, vol. 11, 14 June 2021 (2021-06-14), XP055891972, DOI: 10.3389/fonc.2021.685026 | 10-12 | TECHNICAL FIELDS SEARCHED (IPC) C12Q |
| A | * abstract; figure 6 * * page 9, columns 1-2 * | 1-9,13 | |

-/--

The present search report has been drawn up for all claims

| Place of search | Date of completion of the search | Examiner |
|---|---|---|
| The Hague | 16 February 2022 | Aguilera, Miguel |

EPO FORM 1503 03.82 (P04C01)

Europäisches
Patentamt

European
Patent Office

Office européen
des brevets

## EUROPEAN SEARCH REPORT

**Application Number**

**EP 21 38 2772**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | PEDROSA L: "An immune-metabolic signature correlates with immunesuppressive patterns and with outcome, across tumor types", ANNALS OF ONCOLOGY, vol. 32, suppl. 5, 1 September 2021 (2021-09-01), page S847, XP055892357, DOI: 10.1016/j.annonc.2021.08.1380 * abstract * | | |

TECHNICAL FIELDS
SEARCHED      (IPC)

The present search report has been drawn up for all claims

| Place of search | Date of completion of the search | Examiner |
|---|---|---|
| The Hague | 16 February 2022 | Aguilera, Miguel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 21 38 2772

This annex lists the patent family members relating to the patent documents cited in the above-mentioned European search report.
The members are as contained in the European Patent Office EDP file on
The European Patent Office is in no way liable for these particulars which are merely given for the purpose of information.

16-02-2022

| Patent document cited in search report | | Publication date | Patent family member(s) | | Publication date |
|---|---|---|---|---|---|
| WO 2019090263 | A1 | 09-05-2019 | AU | 2018359967 A1 | 23-04-2020 |
| | | | CA | 3077664 A1 | 09-05-2019 |
| | | | CN | 111213059 A | 29-05-2020 |
| | | | EP | 3707510 A1 | 16-09-2020 |
| | | | JP | 2021502066 A | 28-01-2021 |
| | | | KR | 20200075860 A | 26-06-2020 |
| | | | TW | 201923089 A | 16-06-2019 |
| | | | US | 2020263261 A1 | 20-08-2020 |
| | | | WO | 2019090263 A1 | 09-05-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7473767 B

**Non-patent literature cited in the description**

- **BURTIS C. A. et al.** *Statistical Treatment of Reference Values,* 2008
- **CRISTESCU R. et al.** Pan-tumor genomic biomarkers for PD-1 checkpoint blockade-based immunotherapy. *Science,* 2018, vol. 362, 3593
- **CHAROENTONG P. et al.** Pan-cancer Immunogenomic Analyses Reveal Genotype-Immunophenotype Relationships and Predictors of Response to Checkpoint Blockade. *Cell Rep.,* 03 January 2017, vol. 18 (1), 248-262
- **MARABELLE A. et al.** Association of tumour mutational burden with outcomes in patients with advanced solid tumours treated with pembrolizumab: prospective biomarker analysis of the multicohort, open-label, phase 2 KEYNOTE-158 study. *Lancet Oncol.,* 2020, vol. 21 (10), 1353-1365
- **PARE L. et al.** Association between PD1 mRNA and response to anti-PD1 monotherapy across multiple cancer types. *Ann Oncol.,* 2018, vol. 29 (10), 2121-2128
- **SHITARA K et al.** Efficacy and Safety of Pembrolizumab or Pembrolizumab Plus Chemotherapy vs Chemotherapy Alone for Patients With First-line, Advanced Gastric Cancer: The KEYNOTE-062 Phase 3 Randomized Clinical Trial. *JAMA Oncol.,* 01 October 2020, vol. 6 (10), 1571-1580